# EUROPEAN PATENT APPLICATION

(11) **EP 4 108 761 A1**
(43) Date of publication of application: **28.12.2022**
(21) Application number: 21756895.5
(22) Date of filing: 19.02.2021
(51) Int. Cl.: C12M 3/00, C08F 20/34, C12M 1/00

(54) **METHOD FOR PRODUCTING CELL AGGREGATE**

(30) Priority: 21.02.2020 JP 2020028119
(71) Applicant: Nissan Chemical Corporation, Tokyo 103-6119 (JP); Kake Educational Institution, Okayama-shi, Okayama 700-0005 (JP)
(72) Inventor: SUZUKI Kohei, Funabashi-shi, Chiba 274-0052 (JP); HIROI Yoshiomi, Funabashi-shi, Chiba 274-0052 (JP); IWAI Ryosuke, Okayama-shi, Okayama 700-0005 (JP)
(74) Representative: dompatent von Kreisler Selting Werner - Partnerschaft von Patent- und Rechtsanwälten mbB
(86) International application number: PCT/JP2021/006243
(87) International publication number: WO 2021/167041

(57) **Abstract**

This is to provide a substrate for producing cell aggregates, a method for producing the same, a method for producing cell aggregates, and a method for improving cell utilization efficiency at the time of producing the cell aggregates.

A substrate for producing cell aggregates provided with a plurality of spots which comprises a polymer containing a recurring unit derived from a monomer represented by the following formula (I): 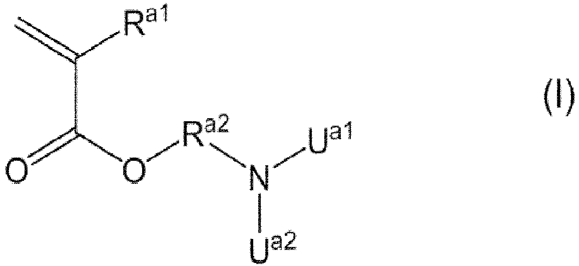 [wherein U^{a1}, U^{a2}, R^{a1} and R^{a2} are as defined in the present specification and claims] on a substrate having an ability to inhibit adhesion of cells, wherein a ratio of a total area of the spots to a surface area of the substrate is 30% or more, a diameter of each spot is 50 to 5,000 µm, and a distance between the spots is 30 to 1,000 µm, a method for producing the same, and a method for producing cell aggregates using such a substrate and a method of improving cell utilization efficiency at the time of producing the cell aggregates.

## Description

### TECHNICAL FIELD

The present invention relates to a substrate for producing cell aggregates, a method for producing the same, a method for producing the cell aggregates, and a method for improving cell utilization efficiency at the time of producing the cell aggregates.

### BACKGROUND ART

It has been reported that cell aggregates (also referred to as a spheroid or a cell mass, etc.) are three-dimensionally cultured, which are cell gathered materials in which cells are self-gathered and aggregated and a living body-like structure is constructed, so that functions of the cells can be maintained for a long term and physiological functions are improved. Therefore, expectations of the cell aggregates for utilization in drug discovery research, or in cell therapy or regenerative therapy is increasing.

Accompanied with this, development of tissue engineering technology that can prepare cell aggregates simply and easily and quickly, and uniformly and a large amount is considered to be an important issue for practical application of regenerative medicine and make drug discovery test efficiency, but in the method of utilizing random aggregation phenomenon of floating cells using a conventional cell low-adhesion culture dish (for example, multi-well plates), only one spheroid is formed per one well, so that there is a problem that it is not excellent in operability and mass productivity.

The present inventors have reported until now that, as a technique relating to cell culture, a coating agent containing a copolymer having a specific anionic group and cationic group can firmly adhered to any of the substrate without selecting the kind thereof, and after adhesion, it becomes a coating film excellent in resistance to an aqueous solvent and shows an excellent ability to inhibit adhesion to biological substances (for example, platelets or fibrinogen, etc.) (for example, see Patent Documents 1 and 2). In addition, they have reported that a cell culture container, which is characterized in that it is coated by the above coating agent, has excellent ability to inhibit adhesion of cells and also excellent in resistance to solvents and radiation, further can be used for non-adhesive culture for a long term (for example, 14 days or longer, for example, 21 days or longer) and suitable for preparation of spheroids (for example, see Patent Documents 3 and 4). Further, the present inventors have also reported about a coating agent which induces spontaneous assembly (self-gathered) of adherent cells and preparation of spheroids using a cell culture container which is characterized in that it is coated by the above coating agent (for example, see PCT/JP2019/32785). However, none of these was satisfactory in terms of operability and mass productivity for producing spheroids.

### PRIOR ART DOCUMENT

### PATENT DOCUMENT

Patent Document 1: WO 2014/196650
Patent Document 2: WO 2016/093293
Patent Document 3: WO 2014/196652
Patent Document 4: WO 2019/107503

### SUMMARY OF THE INVENTION

### PROBLEMS TO BE SOLVED BY THE INVENTION

An object of the present invention is to provide a substrate for producing cell aggregates and a method for producing the same, which are excellent in operability and mass productivity for producing spheroids.

### MEANS TO SOLVE THE PROBLEMS

The present inventors have found that a coating agent which induces spontaneous assembly (self-gathered) of adherent cells is combined with, for example, an inkjet device, and applying it to a substrate having an ability to inhibit adhesion of cells (for example, a cell low-adhesion petri dish) with high-density and in a spot shape whereby almost all of the seeded cells can be adhered to the coating film (spots) and aggregated, so that they have completed the present invention.

That is, the present invention is as follows:
[1] A substrate for producing cell aggregate provided with a plurality of spots which comprises a polymer containing a recurring unit derived from a monomer represented by the following formula (I): 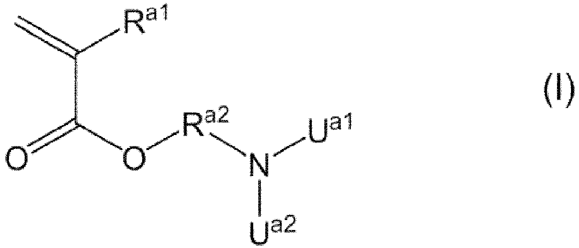 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms]
   on a substrate having an ability to inhibit cell adhesion, wherein a ratio of a total area of the spots to a surface area of the substrate is 30% or more, a diameter of each spot is 50 to 5,000 µm, and a distance between the spots is 30 to 1,000 µm.
[2] The substrate for producing cell aggregates described in [1], wherein cell utilization efficiency is 90% or more.
[3] The substrate for producing cell aggregates described in [1] or [2], wherein the cell is a stem cell.
[4] The substrate for producing cell aggregates described in any one of [1] to [3], wherein the polymer further contains a recurring unit derived from a monomer represented by the formula (II): 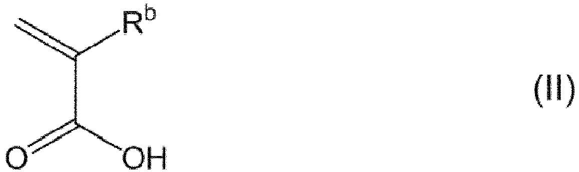 [wherein
   R^{b} represents a hydrogen atom or a linear or branched alkyl group having 1 to 5 carbon atoms].
[5] The substrate for producing cell aggregates described in any one of [1] to [4], wherein the polymer further contains a structural unit derived from a monomer represented by the following formula (III): 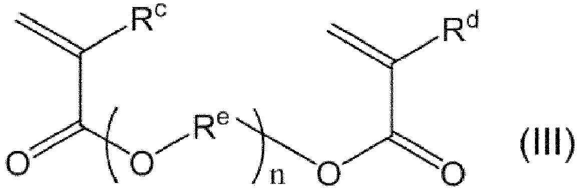 [wherein
   R^{c} and R^{d} each independently represent a hydrogen atom or a linear or branched alkyl group having 1 to 5 carbon atoms, R^{e} represents a linear or branched alkylene group having 1 to 5 carbon atoms, and n represents a number of 1 to 50].
[6] The substrate for producing cell aggregates described in any one of [1] to [5], wherein the substrate having an ability to inhibit cell adhesion comprises a coating film which comprises a copolymer (P) having a recurring unit containing a group represented by the following formula (a) and a recurring unit containing a group represented by the following formula (b): 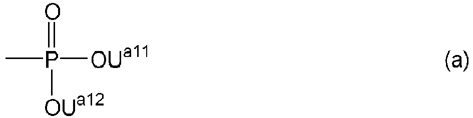 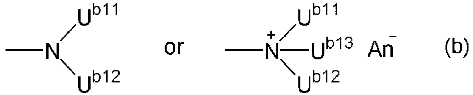 [wherein
   U^{a11}, U^{a12}, U^{b11}, U^{b12} and U^{b13} each independently represent a hydrogen atom or a linear or branched alkyl group having 1 to 5 carbon atoms, An⁻ represents an anion selected from the group consisting of a halide ion, an inorganic acid ion, a hydroxide ion and an isothiocyanate ion]
   at least a part of a surface of the substrate.
[7] A method for producing a substrate for producing cell aggregates which comprises a step of applying a polymer containing a recurring unit derived from a monomer represented by the following formula (I): 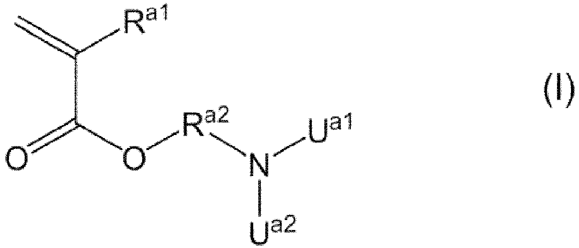 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms] to a substrate having an ability to inhibit cell adhesion in a state of a plurality of spots, and then, drying, wherein a total area of the spots based on a surface area of the substrate is 30% or more, a diameter of each spot is 50 to 5,000 µm, and a distance between the spots is 30 to 1,000 µm.
[8] The method for producing a substrate for producing cell aggregate described in [7], wherein cell utilization efficiency is 90% or more.
[9] The method for producing a substrate for producing cell aggregate described in [7] or [8], wherein the applying step is carried out by an inkjet system.
[10] A method for producing cell aggregates which comprises a step of applying a polymer containing a recurring unit derived from a monomer represented by the following formula (I): 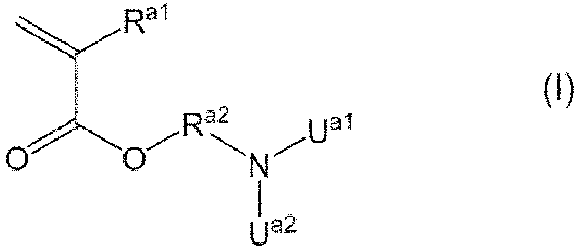 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms] to a substrate having an ability to inhibit cell adhesion in a state of a plurality of spots, and then, a step of seeding cells, wherein a total area of the spots based on a surface area of the substrate is 30% or more, a diameter of each spot is 50 to 5,000 µm, and a distance between the spots is 30 to 1,000 µm.
[11] The method for producing cell aggregate described in [10], wherein cell utilization efficiency is 90% or more.
[12] A method for improving cell utilization efficiency at a time of producing cell aggregates by applying a polymer containing a recurring unit derived from a monomer represented by the following formula (I): 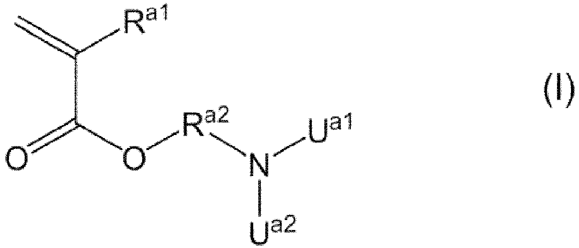 [wherein
   U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms] to a substrate having an ability to inhibit cell adhesion in a state of a plurality of spots, wherein a total area of the spots based on a surface area of the substrate is 30% or more, a diameter of each spot is 50 to 5,000 µm, and a distance between the spots is 30 to 1,000 µm.

### EFFECTS OF THE INVENTION

According to the present invention, without using the conventional multi-well plates, etc., for preparing spheroids, by using a substrate for producing cell aggregates of the present invention which is provided with a plurality of spot state coating film comprising a specific polymer according to the present invention with high density, which induces spontaneous assembly (self-assembly) of adherent cells, on a substrate having an ability to inhibit adhesion of cells, which is a bottom surface of a petri dish or dishes or a surface of a plate, a plurality of spheroids with a uniform size can be formed by one substrate (container) only by seeding cells on the substrate plane surface having the coating film (spots), so that it is excellent in operability and mass productivity. In addition, by using the substrate for producing cell aggregates of the present invention, utilization efficiency of the cells seeded at the time of producing spheroids is also improved. Therefore, according to the present invention, spheroids can be produced simply and easily and efficiently, uniformly and with a large amount.

### BRIEF DESCRIPTION OF THE DRAWINGS

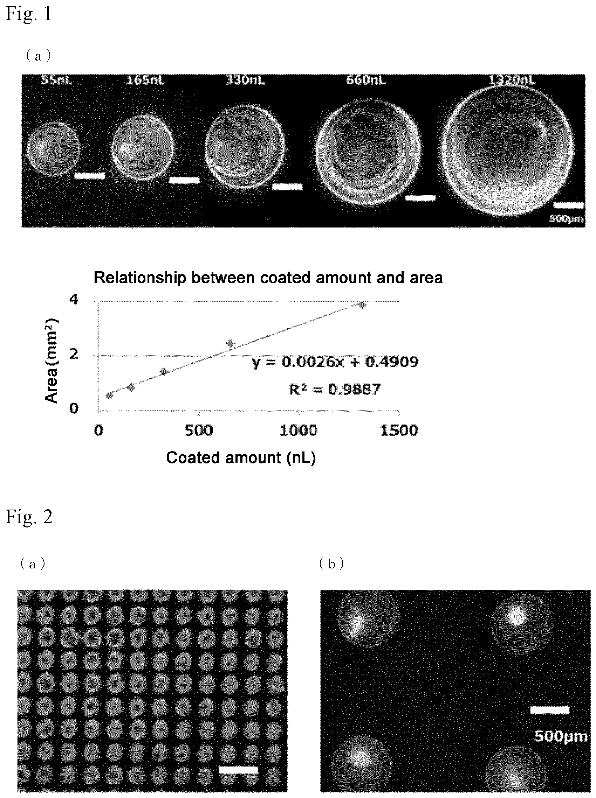

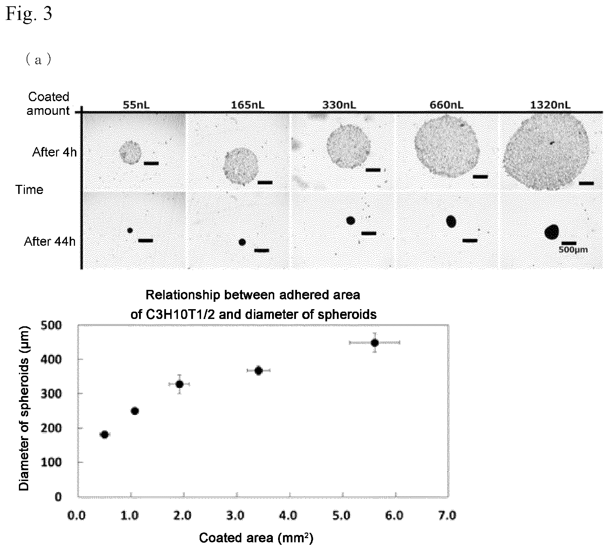

Fig. 1(a) is a fluorescence microscopic image of a petri dish for forming the cell aggregates obtained in Example 1, the portion on which the polymer according to the present invention is coated is shining in a circle, and it is shown that a coating film (spot) can be formed by inkjet. (b) is a graph showing a relationship between an amount of droplets (coated amount) of inkjet and an area of the formed coating film in the production of a petri dish for forming cell aggregates of Example 1, and it is shown that accompanied with increase in the amount of droplets, an area of the spots is increased, that is, a size of the spots can be controlled by the amount of droplets.
Fig. 2(a) is a fluorescence microscopic image of a petri dish for forming the cell aggregates obtained in Example 2, and (b) is a fluorescence microscopic image of a petri dish for forming the cell aggregates obtained in Comparative Example 1. A ratio of the total area of the spots to the bottom surface area of the petri dish was 44% for the former and 15% for the latter.
The upper row of Fig. 3(a) is a microscopic image showing the state (after 4 hours) in which cells are adhered to each spot of a petri dish for forming the cell aggregates in the cell aggregates producing test of Example 3, and the lower row of (a) is a microscopic image of the state (after 44 hours) of the spheroids formed by exfoliation of the adhered cells. (b) is a graph showing a relationship between an area of the spots and a diameter of the spheroids, and it is shown that accompanied with increase in the amount of the spots, the diameter of the spheroids is increased, that is, a size of the spheroids can be controlled by the amount of droplets.

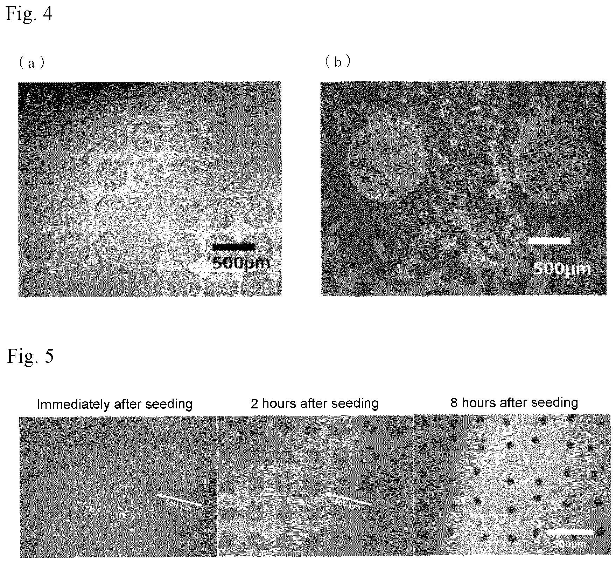

Fig. 4(a) is a microscopic image of the state in which cells are adhered to each spot of a petri dish for forming the cell aggregates having spots with high-density in the cell adhesion confirmation test of Example 4, and (b) is a microscopic image of the state in which cells are adhered to each spot of a petri dish for forming the cell aggregates having spots with low-density in the cell adhesion confirmation test of Comparative Example 2. A ratio of the total area of the spots to the bottom surface area of the petri dish was 52% for the former and 15% for the latter.
Fig. 5 is a part of microscopic images in which the state of the cells (immediately after seeding) seeded to a petri dish for forming cell aggregates in the cell aggregates production test of Example 5 is photographed with a time-lapse, which are adhered to each spot of the petri dish with a lapse of time (after 2 hours), form the cell aggregates (after 8 hours).

### EMBODIMENTS TO CARRY OUT THE INVENTION

### [Substrate for producing cell aggregates and method for producing the same] (Polymer)

The substrate for producing cell aggregate of the present invention is provided with a plurality of spots comprising a polymer on a substrate having an ability to inhibit adhesion of cells. In the present invention, the "spot" means a substantially circular coating film formed on the substrate by a polymer having a diameter of 50 to 5,000 µm. The polymer constituting the spot portion contains a recurring unit derived from a cationic monomer represented by
the following formula (I): 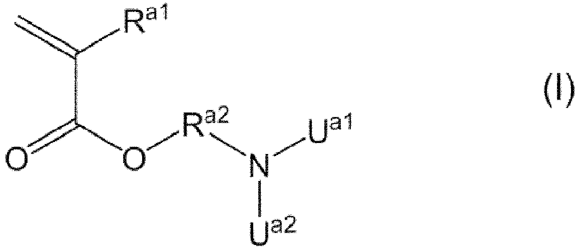 [wherein
U^{a1}, U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms].

The above-mentioned polymer is preferably a copolymer containing, together with the cationic monomer represented by the above-mentioned formula (I), a recurring unit derived from an anionic monomer represented by the following formula (II): 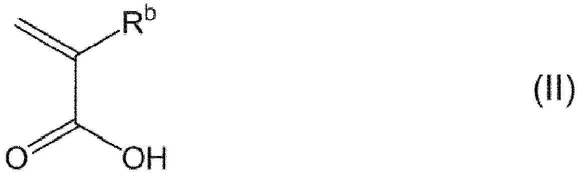 [wherein
R^{b} represents a hydrogen atom or a linear or branched alkyl group having 1 to 5 carbon atoms].

In the present specification, unless otherwise specifically defined, as the "linear or branched alkyl group having 1 to 5 carbon atoms", there may be mentioned, for example, a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group.

R^{a1} and R^{b} are preferably each independently selected from a hydrogen atom and a methyl group.

U^{a1} and U^{a2} are preferably each independently selected from a hydrogen atom, a methyl group, an ethyl group, an n-propyl group, an isopropyl group and an n-butyl group, preferably a methyl group or an ethyl group, and most preferably a methyl group.

In the present specification, unless otherwise specifically defined, as the "linear or branched alkylene group having 1 to 5 carbon atoms", there may be mentioned, for example, a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group, a 1-ethyl-trimethylene group, etc. Among these, as R^{a2}, it is preferably selected from an ethylene group and a propylene group.

Accordingly, as the cationic monomer represented by the above-mentioned formula (I), there may be mentioned 2-N,N-dimethylaminoethyl methacrylate, N-isopropylacrylamide, etc., and preferably 2-N,N-dimethylaminoethyl methacrylate. As the anionic monomer represented by the above-mentioned formula (II), there may be mentioned acrylic acid, methacrylic acid, etc., and preferably methacrylic acid.

A molar ratio of the unit derived from the monomer represented by the formula (I)/the unit derived from the monomer represented by the formula (II) in the above-mentioned polymer is 100/0 to 50/50. It is preferably 98/2 to 50/50. It is more preferably 98/2 to 60/40, and particularly preferably 98/2 to 70/30.

If the molar ratio of the formula (II) is 51 or more, the anionic property of the polymer becomes excessive and adhesive force of the cells is lowered.

The above-mentioned polymer preferably contains, together with the monomers represented by the formula (I)/the formula (II), further a structural unit derived from a monomer having two or more carbon-carbon unsaturated bonds. The monomer having two or more carbon-carbon unsaturated bonds is specifically a monomer having two or more carbon-carbon double bonds, and there may be mentioned, for example, a polyfunctional acrylate compound, a polyfunctional acrylamide compound, a polyfunctional polyester or an isoprene compound, etc. The structural units derived from these monomers can exist as crosslinking structure between the polymers.

By introducing the above-mentioned monomer having two or more carbon-carbon unsaturated bonds, the polymer becomes high molecular weight and the molecular weight distribution is expanded so that adhesiveness and coatability of the film to the substrate can be improved.

Preferred specific examples may be mentioned monomers represented by the following formulae (III) to (V).

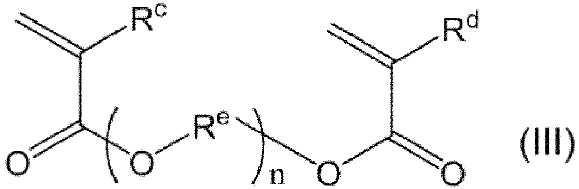

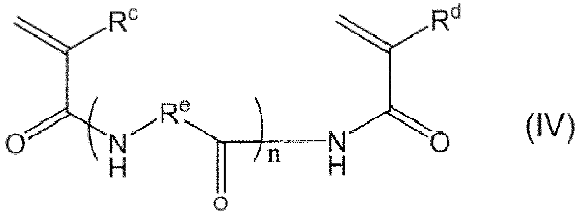

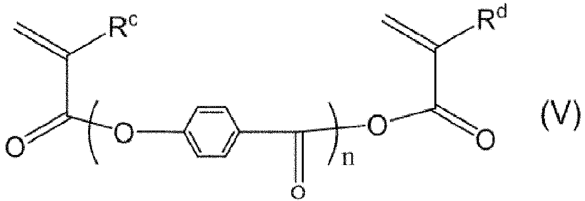

In the formula, R^{c} and R^{d} each independently represent a hydrogen atom or a linear or branched alkyl group having 1 to 5 carbon atoms, R^{e} represents a linear or branched alkylene group having 1 to 5 carbon atoms, and n represents a number of 1 to 50. Among these, it is preferably a monomer represented by the formula (III).

A molar ratio of the monomer represented by the formulae (III) to (V) based on the whole polymer mentioned above is preferably 0 to 5%. It is further preferably 0 to 3%.

If the molar ratio of the formulae (III) to (V) is 5% or more, there is a fear of making difficult to produce due to gelation during production by becoming higher molecular weight due to excessive crosslinking.

R^{c} and R^{d} are each independently and preferably selected from a hydrogen atom and a methyl group.

R^{e} is preferably selected from a methylene group, an ethylene group and a propylene group, and most preferably an ethylene group.
n is a number of 1 to 50, n is preferably a number of 1 to 30, and n is preferably a number of 1 to 10.

A difference between the value of mol% occupied by the monomer represented by the formula (II) based on the above-mentioned whole polymer and the value of mol% occupied by the monomer represented by the formula (II) based on the total amount of the monomers charged during the preparation step is 0 to 10 mol%. The polymer according to the present invention has a small difference between the monomer charging ratio and the measured value of the produced polymer, by the production method mentioned later, and is 0 to 10 mol%. It is further preferably 0 to 8 mol%.

A number average molecular weight (Mn) of the above-mentioned polymer is 20,000 to 2,000,000, and further preferably 50,000 to 1,000,000.

A ratio (Mw/Mn) of the weight average molecular weight (Mw) and the above-mentioned number average molecular weight (Mn) of the above-mentioned polymer is 1.01 to 10.00, preferably 1.2 to 8.0, more preferably 2.0 to 7.0, and particularly preferably 3.0 to 6.0.

The weight average molecular weight (Mw) and the number average molecular weight (Mn) can be obtained, for example, by Gel Filtration Chromatography described in Examples.

By utilizing the substrate for producing cell aggregates of the present invention which is provided with a plurality of spots comprising the above-mentioned polymer on a substrate having an ability of inhibiting adhesion of cells, it is possible to produce a plurality of the cell aggregates with one container at once by adhering cells to the plurality of the spot portions and then peeling off the same. Incidentally, the cell aggregate refers to a structural material formed as a result of aggregation of the cells, and a shape thereof is not limited to a spherical shape or a ring shape, etc. The present invention has a merit in the point that it is possible to adjust a size of the cell aggregates according to the regulation of the adhesion area (cell aggregates having an optional size can be produced), etc., as compared with the cell aggregates produced on a conventional cell low-adhesion plate by non-adhesive culture.

### (Method for producing polymer)

The polymer according to the present invention can be produced by the thermal polymerization method. For example, the monomer of the above-mentioned formula (I) is dissolved in an organic solvent, a radical polymerization initiator is added thereto, and then, if necessary, the monomer of the above-mentioned formula (II), and further, if necessary, the monomer having two or more carbon-carbon unsaturated bonds (the monomer(s) represented by the formulae (III) to (V), etc.) is/are added thereto to prepare a mixture, and then, sufficiently stirring to make the mixture uniform, and the mixture is stirred while nitrogen flowing by raising the temperature to, for example, 51°C or higher, for example, 51 to 180°C, 51 to 150°C, 51 to 130°C, 51 to 100°C, for example, a reflux temperature of the solvent (for example, 66 to 85°C in the case of tetrahydrofuran), and for example, 1 to 48 hours to obtain a polymer. The obtained polymer may be purified by reprecipitation or dialysis.

In one embodiment, it can be prepared by the producing method which comprises the steps of dissolving the monomer of the above-mentioned formula (I) in a solvent, adding a polymerization initiator, and if necessary, the monomer of the above-mentioned formula (II) thereto, and reacting (polymerizing) it with a total concentration of both compounds of 0.01% by mass to 40% by mass in the solvent.

As the organic solvent used in the above-mentioned polymerization, there may be mentioned, for example, an ether solvent such as tetrahydrofuran, 1,4-dioxane, etc., an aliphatic alcohol solvent having 1 to 4 carbon atoms such as methanol, ethanol, isopropanol, etc., an aromatic hydrocarbon solvent such as toluene, etc., or a mixed solvent thereof.

In order to efficiently proceed with the polymerization reaction, it is desirable to use a radical polymerization initiator. Examples of the radical polymerization initiators, there may be mentioned azo polymerization initiators such as dimethyl 1,1'-azobis(1-cyclohexanecarboxylate) (VE-073, available from FUJIFILM Wako Pure Chemical Corporation), 2,2'-azobis(2,4-dimethylvaleronitrile) (V-65, available from FUJIFILM Wako Pure Chemical Corporation), 2,2'-azobis(isobutyronitrile) (AIBN, available from FUJIFILM Wako Pure Chemical Corporation), 2,2'-azobis[N-(2-carboxyethyl)-2-methylpropionamidine] n hydrate (VA-057, available from FUJIFILM Wako Pure Chemical Corporation), 2,2'-(N-butyl-2-methylpropionamide) (VAm-110, available from FUJIFILM Wako Pure Chemical Corporation), etc.

As an amount of the polymerization initiator to be added, it is 0.05% by mass to 5% by mass based on the total weight of the monomers used for the polymerization.

Use of the polymerization initiator makes it possible not only to improve efficiency of the polymerization reaction, but also to adjust the physical characteristics of the polymer by modifying the terminal functional group.

For example, the polymer according to the present invention may introduce a functional molecule by modification of a terminal functional group within the range which does not impair the object thereof. A typical example of the functional molecule is a fluorescently labeled molecule, and a reagent for introducing such a molecule is commercially available under a name, for example, Alexa Fluor (trademark), etc.

### (Spot)

A plurality of spots (coating film) comprising the above-mentioned polymer provided by the substrate for producing cell aggregates of the present invention is characterized in that a ratio of the total area of the spots to the surface area of the substrate is 30% or more, and a diameter of each spot is 50 to 5,000 µm, and a distance between the spots is 30 to 1,000 µm. The ratio of the total area of the spots to the surface area of the substrate, the diameter of each spot and the distance between the spots can be optionally selected from the predetermined range depending on the kinds of the cells and the substrate to be used, the desired size of the cell aggregates, etc., and the ratio of the total area of the spots to the surface area of the substrate is preferably 30% or more, 40% or more and 50% or more, and preferably 99% or less, the diameter of each spot is 50 to 5,000 µm, and preferably 300 to 3,000 µm, and the distance between the spots is 30 to 1,000 µm, and preferably 100 to 500 µm. In the present invention, a plurality of spheroids having uniform size can be formed with one substrate (container) at once by arranging independent regions (spots) with such a high-density, and preferably regularly, having a micro-size capable of adhering cells, on a substrate having an ability to inhibit adhesion of cells.

Also, in the substrate for producing cell aggregates of the present invention, almost all of the seeded cells can be adhered to the spot and aggregated. Specifically, in the substrate for producing cell aggregates of the present invention, cell utilization efficiency is 90% or more, preferably 95% or more, and more preferably 99% or more. Incidentally, the cell utilization efficiency is a value shown in a percentage by dividing the number of adherent cells (for example, a value of subtracting the number of not adherent cells contained in the medium removed from the substrate after incubation treatment from the number of seeded cells) by the number of the seeded cells, after seeding cells to the substrate for producing cell aggregates of the present invention and a predetermined incubation operation.

A plurality of spots (coating film) comprising the above-mentioned polymer, which are provided with the substrate for producing cell aggregates of the present invention, are formed by the steps of applying the above-mentioned polymer to the substrate having an ability to inhibit adhesion of cells in a spot shape, and then, drying.

The step of applying the above-mentioned polymer to the substrate may be carried out using a coating film-forming agent containing the above-mentioned polymer. The coating film-forming agent can be produced by mixing a water-containing solution with the above-mentioned polymer by the method known *per se* to produce the above-mentioned coating film-forming agent.

The water-containing solution may be mentioned water, a salt-containing aqueous solution such as a physiological saline or a phosphate buffer solution, etc., or a mixed solvent in which water or the salt-containing aqueous solution and an alcohol are combined. As the alcohol, there may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (=neopentyl alcohol), 2-methyl-1-propanol, 2-methyl-1-butanol, 2-methyl-2-butanol (=t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol, and these may be used alone or may be used a mixed solvent in combination thereof.

Further, to the coating film-forming agent may be added, in addition to the above-mentioned polymer and the solvent, other substance(s) within the range which do not impair the properties of the resulting coating film, if necessary. As the other substance(s), there may be mentioned pH adjusting agents, crosslinking agents, preservatives, surfactants, primers that enhance adhesiveness to the substrate, fungicides and saccharides, etc.

### (Substrate)

As the substrate to be coated with the above-mentioned coating film-forming agent, there may be mentioned, for example, petri dishes or dishes, plates and trays which are generally used for culture of cells, and "on the substrate" means a surface that comes into contact with cells or cell culture, etc., in these, and for example, in the case of petri dish or dishes, it means the bottom surface.

Also, the material of the substrate may be mentioned, for example, glass, a metal, a metal containing compound or a semi-metal containing compound, activated charcoal or a resin. The metal may be mentioned a typical metal: (an alkali metal: Li, Na, K, Rb, Cs; an alkaline earth metal: Ca, Sr, Ba, Ra), a magnesium group element: Be, Mg, Zn, Cd, Hg; an aluminum group element: Al, Ga, In; a rare earth element: Y, La, Ce, Pr, Nd, Sm, Eu; a tin group element: Ti, Zr, Sn, Hf, Pb, Th; an iron group element: Fe, Co, Ni; a vanadium group element: V, Nb, Ta, a chromium group element: Cr, Mo, W, U; a manganese group element: Mn, Re; a noble metal: Cu, Ag, Au; and a platinum group element: Ru, Rh, Pd, Os, Ir, Pt, etc. The metal containing compound or the semi-metal containing compound may be mentioned, for example, ceramics comprising a metal oxide as a basic component, which are a sintered body baked by a heat treatment at a high temperature, a semiconductor such as silicon, an inorganic solid material including a molded product of an inorganic compound such as a metal oxide or a semi-metal oxide (silicon oxide, alumina, etc.), a metal carbide or a semi-metal carbide, a metal nitride or a semi-metal nitride (silicon nitride, etc.), a metal boride or a semi-metal boride, etc., aluminum, nickel-titanium and stainless (SUS304, SUS316, SUS316L, etc.).

As the resin, it may be either of a natural resin or a derivative thereof, or a synthetic resin, as the natural resin, there may be mentioned, for example, cellulose, cellulose triacetate (CTA), nitrocellulose (NC), cellulose to which dextran sulfate has been fixed, etc., and as the synthetic resin, there may be preferably used polyacrylonitrile (PAN), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), polyvinyl chloride (PVC), polytetrafluoroethylene (PTFE), ultra-high molecular weight polyethylene (UHPE), polydimethylsiloxane (PDMS), acrylonitrile-butadiene-styrene resin (ABS) or Teflon (Registered Trademark). In the production of the substrate for producing cell aggregates of the present invention, at the time of applying the polymer to the substrate, it is not necessary to treat it at a high temperature, so that a resin having low heat resistance, etc., can be also applied.

A material(s) of the substrate may be one kind or a combination of two or more kinds. Among these materials, it is preferably glass, silicon, silicon oxide, polystyrene (PS), polypropylene(PP), polyether sulfone (PES), polyethylene terephthalate (PET), polycarbonate (PC), polyvinyl chloride (PVC), Teflon (Registered Trademark), cycloolefin polymer (COP), polydimethylsiloxane (PDMS) or stainless (SUS304, SUS316, SUS316L, etc.) alone, or a combination selected from these, and particularly preferably glass, polystyrene (PS), polypropylene (PP), stainless (SUS304, SUS316, SUS316L, etc.) or polydimethylsiloxane (PDMS).

In the applying step, with regard to the plurality of spots to be formed, it is carried out so that the ratio of the total area of the spots to the surface area of the substrate is 30% or more, and the diameter of each spot is 50 to 5,000 µm and the distance between the spots is to be 30 to 1,000 µm, and as the system of the applying, there may be employed, for example, an inkjet method, a screen printing method, a slit coating method, a roll-to-roll method, etc., and preferably carried out by a printing technique of the inkjet method. The inkjet device and the inkjet head, etc., used in the inkjet method are not particularly limited, and can be appropriately selected from commercially available products according to the physical properties of the above-mentioned polymer or the coating film-forming agent, the amount of droplets, etc.

Also, the coating film on the substrate obtained by such a method can be used as a substrate for producing cell aggregates, after the applying step, without subjecting to the drying step as such, or after washing using water or a medium of the sample applied to cell culture (for example, water, buffer solution, medium, etc.).

The substrate may be subjected to a drying step. The drying step is carried out under atmosphere or under vacuum, preferably at a temperature in the range of -200°C to 200°C. According to the drying step, by removing the solvent in the above-mentioned coating film-forming agent, it completely adheres to the substrate.

The coating film can be also formed, for example, by drying at room temperature (10°C to 35°C, preferably 20°C to 30°C, for example, 25°C), but in order to form the coating film more quickly, for example, it may be dried at 40°C to 50°C. If the drying temperature is lower than -200°C, a refrigerant which is not common must be used so that it is not versatile, and it takes a long time to dry due to solvent sublimation so that efficiency is bad. If the drying temperature exceeds 200°C, thermal decomposition of the polymer occurs. A more preferable drying temperature is 10°C to 180°C, and a more preferable drying temperature is 20°C to 150°C.

The coating film (spot) of the substrate for producing cell aggregates of the present invention is produced through the above simple and easy steps.

Also, in order to eliminate impurities, unreacted monomers, etc., remained in the coating film, a step of washing with at least one kind of a solvent selected from water and an aqueous solution containing an electrolyte(s) may be carried out. Washing is desirably running water washing or ultrasonic washing, etc. The above-mentioned water and an aqueous solution containing an electrolyte(s) may be a material heated in the range of, for example, 40°C to 95°C. The aqueous solution containing an electrolyte(s) is/are preferably PBS, physiological saline (containing only sodium chloride), Dulbecco's phosphate buffered physiological saline, Tris buffered physiological saline, HEPES buffered physiological saline and Veronal buffered physiological saline, and particularly preferably PBS. After fixing, the coating film does not dissolve out and remains firmly fixed to the substrate even when it is washed with water, PBS and alcohol, etc.

Regarding the film thickness of the coating film of the substrate for producing cell aggregates of the present invention, the maximum film thickness and the minimum film thickness are in the range of 1 to 1,000 nm, and preferably in the range of 5 to 500 nm.

The substrate is a material which is subjected to a treatment of inhibiting adhesion of cells before the applying and drying step of the above-mentioned coating film. The substrate having an ability to inhibit adhesion of cells can be produced, for example, through a step of applying a known composition for forming a coating film having an ability of inhibiting adhesion of cells (for example, described in WO2014/196650 and WO2016/093293). As the composition for forming a coating film having an ability of inhibiting adhesion of cells, preferred is a composition for forming a coating film which contains a copolymer (P) having a recurring unit containing the group represented by the following formula (a) and a recurring unit containing the group represented by the following formula (b): 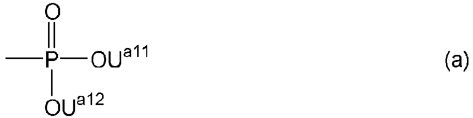 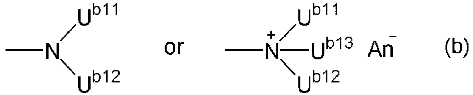 [wherein
U^{a11}, U^{a12}, U^{b11}, U^{b12} and U^{b13} each independently represent a hydrogen atom or a linear or branched alkyl group having 1 to 5 carbon atoms, An⁻ represents an anion selected from the group consisting of a halide ion, an inorganic acid ion, a hydroxide ion and an isothiocyanate ion]
and a solvent, it is preferable to contain steps of applying it to the surface of a container or a substrate and drying.

The copolymer (P) according to the present invention may be copolymerized with, as a further optional third component, an ethylenically unsaturated monomer, a polysaccharide or a derivative thereof. Examples of the ethylenically unsaturated monomer may be mentioned one kind or two or more kinds of ethylenically unsaturated monomers selected from the group consisting of a (meth)acrylic acid and an ester thereof; vinyl acetate; vinylpyrrolidone; ethylene; vinyl alcohol; and a hydrophilic functional derivative thereof. Examples of the polysaccharide or a derivative thereof may be mentioned a cellulose-based polymer such as hydroxyalkyl cellulose (for example, hydroxyethyl cellulose or hydroxypropyl cellulose), etc., starch, dextran and curdlan.

The hydrophilic functional derivative refers to an ethylenically unsaturated monomer having a hydrophilic functional group or structure. Examples of the hydrophilic functional group or structure may be mentioned a betaine structure; an amide structure; an alkylene glycol residue; an amino group; and a sulfinyl group, etc.

The betaine structure means a monovalent or a divalent group of a compound having an amphoteric center of a quaternary ammonium type cation structure and an acidic anion structure and may be mentioned, for example, a phosphorylcholine group: 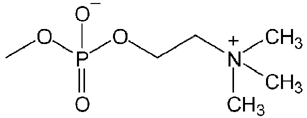 Examples of the ethylenically unsaturated monomer having such a structure may be mentioned 2-methacryloyloxyethyl phosphorylcholine (MPC), etc.

The amide structure means a group represented by the following formula: 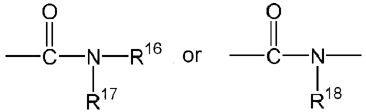 [here, R¹⁶, R¹⁷ and R¹⁸ are each independently a hydrogen atom or an organic group (for example, a methyl group, a hydroxymethyl group or a hydroxyethyl group, etc.)]. Examples of the ethylenically unsaturated monomer having such a structure may be mentioned (meth)acrylamide, N-(hydroxymethyl) (meth)acrylamide, etc. Further, the monomer or the polymer having such a structure is disclosed in, for example, JP 2010-169604A, etc.

The alkylene glycol residue means an alkyleneoxy group (-Alk-O-) which remains after a hydroxyl group(s) at one side terminal or both terminals of the alkylene glycol (HO-Alk-OH; here, Alk is an alkylene group having 1 to 10 carbon atoms) is/are subjected to condensation reaction with other compound(s), and a poly(alkyleneoxy) group in which alkyleneoxy units are repeated is also included. Examples of the ethylenically unsaturated monomer having such a structure may be mentioned 2-hydroxyethyl (meth)acrylate, methoxypolyethylene glycol (meth)acrylate, etc. Further, the monomer or the polymer having such a structure is disclosed in, for example, JP 2008-533489A, etc.

The amino group means a group represented by the formula: -NH₂, -NHR¹⁹ or -NR²⁰R²¹ [here, R¹⁹, R²⁰ and R²¹ are each independently an organic group (for example, a linear or branched alkyl group having 1 to 5 carbon atoms, etc.)]. In the amino group of the present invention, a quaternalized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 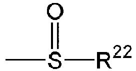 [here, R²² is an organic group (for example, an organic group having 1 to 10 carbon atoms, and preferably an alkyl group having 1 to 10 carbon atoms and having one or more hydroxyl groups, etc.)].
As a polymer having such a structure, there may be mentioned a copolymer disclosed in the publication of JP 2014-48278A, etc.

The linear or branched alkyl group having 1 to 5 carbon atoms is the same as mentioned above.

As the above-mentioned composition for forming a coating film, for example, a composition for forming a coating film described in WO2014/196650 can be used.

The coating method of the above-mentioned composition for forming a coating film is not particularly limited, and a usual coating method such as spin coating, dip coating, solvent casting method, etc., is used.

The drying step of the above-mentioned coating film is carried out under atmosphere or under vacuum at a temperature in the range of -200°C to 180°C. According to the drying step, the solvent in the above-mentioned composition for forming a coating film is removed, and the formula (a) and the formula (b) in the above-mentioned copolymer form an ionic bond and are completely adhered to the substrate.

The above-mentioned coating film can be also formed, for example, by drying at room temperature (10°C to 35°C, for example, 25°C), but in order to form the coating film more quickly, for example, it may be dried at 40°C to 50°C. In addition, a drying step at an extremely low temperature to a low temperature (around -200°C to -30°C) by a freeze-drying method may be used. Freeze-drying is called to as vacuum freeze-drying, which is a method of usually cooling what is desired to be dried with a refrigerant and removing the solvent by sublimation in a vacuum state. A general refrigerant used in freeze-drying may be mentioned a mixed medium of dry ice and methanol (-78°C), liquid nitrogen (-196°C), etc.

If the drying temperature is -200°C or lower, a refrigerant which is not common must be used so that it is not versatile, and it takes a long time to dry due to solvent sublimation so that efficiency is bad. If the drying temperature is 200°C or higher, the ionic bonding reaction on the surface of the coating film proceeds excessively and the surface loses its hydrophilicity whereby the ability to inhibit adhesion of biological substances is not exhibited. A more preferable drying temperature is 10°C to 180°C, and a more preferable drying temperature is 25°C to 150°C.

After drying, it is desirable to carry out washing such as running water washing or ultrasonic washing, etc., with one or more solvents selected from water and an aqueous solution containing an electrolyte(s) in order to eliminate impurities, unreacted monomers, etc., remained on the coating film, and further to adjust the ion balance of the copolymer in the film. The above-mentioned water and an aqueous solution containing an electrolyte(s) may be a material heated in the range of, for example, 40°C to 95°C. The aqueous solution containing an electrolyte(s) is/are preferably PBS, physiological saline (containing only sodium chloride), Dulbecco's phosphate buffered physiological saline, Tris buffered physiological saline, HEPES buffered physiological saline and Veronal buffered physiological saline, and particularly preferably PBS. After fixing, the coating film does not dissolve out and remains firmly fixed to the substrate even when it is washed with water, PBS and alcohol, etc. Even when a biological substance is attached to the formed coating film, it can be easily removed by washing with water, etc., thereafter, and the surface of the substrate on which the above-mentioned coating film is formed has an ability to inhibit adhesion of the biological substance.

A film thickness of the above-mentioned coating film is preferably 5 to 1,000 nm, and further preferably 5 to 500 nm.

Also, as the above-mentioned substrate, s commercially available cell culture dish subjected to cell low-adhesion treatment, or a cell culture container having an ability to inhibit cell adhesion may be used, and for example, a cell culture container described in JP 2008-61609A can be used but it is not limited to this.

Having an ability to inhibit cell adhesion means that, for example, a relative absorbance (WST O.D. 450 nm) (%) ((absorbance of Example (WST O.D.450 nm))/(absorbance of Comparative Example (WST O.D. 450 nm))) when compared with no coating film or without cell low-adhesion treatment by the fluorescence microscope carried out by the method described in Example of WO2016/093293 is 50% or less, preferably 30% or less, and further preferably 20% or less.

### (Cell)

A cell in the present invention is the most basic unit constituting an animal or a plant, and has a cytoplasm and various kinds of organelles inside the cell membrane as its elements. At this time, the nucleus containing DNA may or may not be contained inside the cell. For example, the animal-derived cells in the present invention include germ cells such as sperm and egg cells, etc., somatic cells constituting the living body, stem cells (pluripotent stem cells, etc.), precursor cells, cancer cells separated from the living body, cells separated from the living body and have acquired immortalization ability and are stably maintained in vitro (cell lines), cells separated from the living body and genetic modification has artificially been done, and cells separated from the living body and nuclei of which have artificially been exchanged, etc. Examples of the somatic cells constituting the living body are not limited to the following, and include fibroblast, bone marrow cells, B lymphocytes, T lymphocytes, neutrophils, red blood cells, platelets, macrophages, monocytes, bone cells, bone marrow cells, pericytes, dendritic cells, keratinocytes, fat cells, mesenchymal cells, epithelial cells, epidermal cells, endothelial cells, vascular endothelial cells, hepatic parenchymal cells, cartilage cells, cumulus cells, neural cells, glial cells, neurons, oligodendrocytes, microglia, astroglial cells, heart cells, esophagus cells, muscle cells (for example, smooth muscle cells or skeletal muscle cells), pancreatic beta cells, melanocytes, hematopoietic precursor cells (for example, CD34-positive cells derived from umbilical cord blood), and mononuclear cells, etc. The somatic cells include, for example, cells collected from an optional tissue such as skin, kidney, spleen, adrenal, liver, lung, ovary, pancreas, uterus, stomach, colon, small intestine, large intestine, bladder, prostate, testis, thoracic gland, muscle, connective tissue, bone, cartilage, blood vessel tissue, blood (including umbilical cord blood), bone marrow, heart, eye, brain or nerve tissue, etc. The stem cells are cells that have the ability to replicate themselves and differentiate into other multi-lineage cells, and examples thereof include, but not limited to the following, embryonic stem cells (ES cell), embryonic tumor cells, embryonic germline stem cells, induced pluripotent stem cells (iPS cell), neural stem cells, hematopoietic stem cells, mesenchymal stem cells, liver stem cells, pancreatic stem cells, muscle stem cells, germline stem cells, intestinal stem cells, cancer stem cells, hair follicle stem cells, etc. As the pluripotent stem cells, among the above-mentioned stem cells, there may be mentioned ES cells, embryonic germline stem cells and iPS cells. The precursor cells are cells in the process of differentiating from the above-mentioned stem cells into specific somatic cells or germ cells. The cancer cells are cells that are derived from somatic cells and have acquired infinite proliferative capacity. The cell line is a cell that has acquired infinite proliferative capacity by artificial manipulation *in vitro.* Among these, adherent cells that survive and proliferate by adhering to the scaffold are preferable, and stem cells are more preferable.

### [Method for producing cell aggregates]

The method for producing cell aggregates of the present invention is a method which comprises a step of applying a polymer containing a recurring unit derived from a monomer represented by the following formula (I):

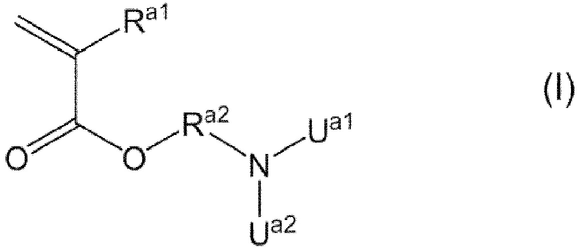

### [wherein

U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms] in a plurality of spot states on a substrate having an ability to inhibit adhesion of cells, then, a step of seeding cells, wherein a total area of the spots based on a surface area of the substrate is 30% or more, a diameter of each spot is 50 to 5,000 µm, and a distance between the spots is 30 to 1,000 µm, and for example, a method for producing cell aggregates carried out by the method described in Examples.

The above-mentioned polymer is preferably a copolymer which contains, together with a recurring unit derived from a monomer represented by the formula (I), a recurring unit derived from a monomer represented by the following formula (II): 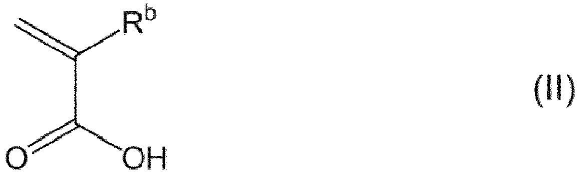 [wherein
R^{b} represents a hydrogen atom or a linear or branched alkyl group having 1 to 5 carbon atoms].

The cells seeded by the above-mentioned method are as described above, and are preferably adherent cells and more preferably stem cells. As others, details and preferred embodiments of the substrate, the polymer, etc., used in the method for producing cell aggregates of the present invention are as described in the above-mentioned [Substrate for producing cell aggregates and method for producing the same].

### [Method for improving cell utilization efficiency at the time of producing cell aggregates]

The method for improving cell utilization efficiency at the time of production of the cell aggregates of the present invention is a method in which a polymer having a recurring unit derived from a monomer represented by the following formula (I): 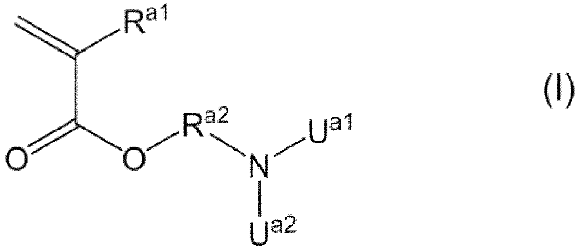 [wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms] in a plurality of spot states on a substrate having an ability to inhibit adhesion of cells, wherein a total area of the spots based on a surface area of the substrate is 30% or more, a diameter of each spot is 50 to 5,000 µm, and a distance between the spots is 30 to 1,000 µm.

Details and preferred embodiments of the substrate, the polymer, etc., used in the method for improving cell utilization efficiency at the time of production of the cell aggregates of the present invention are the same as described in the above-mentioned [Substrate for producing cell aggregates and method for producing the same].

### EXAMPLES

Hereinafter, the present invention will be explained in more detail by referring to Examples, but the present invention is not limited to these.

### <Measurement method of molecular weight>

The weight average molecular weight shown in the following Synthetic Examples is the result by Gel Filtration Chromatography (hereinafter abbreviated to as GFC).

### (Measurement conditions)

- Device: HLC-8320GPC (manufactured by Tosoh Corporation)
- GFC column: TSKgel G 6000 + 3000 PWXL-CP
- Flow rate: 1.0 mL/min
- Eluent: Water/organic mixed solvent containing a salt
- Column temperature: 40°C
- Detector: RI
- Injection concentration: Polymer solid content of 0.05% by mass
- Injection amount: 100 µL
- Calibration curve: Cubic approximation curve
- Standard sample: Polyethylene oxide (available from Agilent Technologies) × 10 kinds

### <Synthetic Example 1> Production (1) of polymer used for coating film-forming agent of cell culture by thermal polymerization

To 10.00 g of 2-(dimethylamino)ethyl methacrylate (available from Tokyo Chemical Industry Co., Ltd.) was added 41.94 g of tetrahydrofuran, and the mixture was sufficiently dissolved. Then, 0.01 g of dimethyl 1,1'-azobis(1-cyclohexanecarboxylate) (VE-073, available from FUJIFILM Wako Pure Chemical Corporation), 0.48 g of methacrylic acid (available from Tokyo Chemical Industry Co., Ltd.) and 0.21 g of polyethylene glycol dimethacrylate (n≒4) (available from Tokyo Chemical Industry Co., Ltd.) were added to the above-mentioned tetrahydrofuran solution while maintaining the temperature of the mixture to 20°C or lower. A mixture containing all of the above, which had been sufficiently stirred and became uniform, was charged in a three-necked flask equipped with a cooling tube, subjected to nitrogen flowing, and the temperature was raised to the reflux temperature while stirring. By heating and stirring the mixture in the state maintaining the above-mentioned environment for 24 hours, a polymer was obtained as a reaction product. The reaction product was reprecipitated with hexane, which is a poor solvent, and the precipitate was collected by filtration and dried under reduced pressure. The obtained powder was dissolved in pure water and the solution was transferred to a dialysis tube. Dialysis was carried out for 72 hours to purify the reaction product.

The solution containing the reaction product was filtered through a 1.0 µm filter (manufactured by AS ONE Corporation, model number: SYGF0605MNXX104) made of a glass fiber, and the obtained filtrate was freeze-dried to obtain a polymer. The weight average molecular weight of this polymer according to GFC was 660,000, and the polydispersity was 3.8 (hereinafter, it is referred to as "Synthetic Example polymer 1").

### <Synthetic Example 2> Production (2) of polymer used for coating film-forming agent of cell culture by thermal polymerization

To 10.00 g of 2-(dimethylamino)ethyl methacrylate (available from Tokyo Chemical Industry Co., Ltd.) was added 31.46 g of ethanol, and the mixture was sufficiently dissolved. Then, 0.01 g of dimethyl 1,1'-azobis(1-cyclohexanecarboxylate) (VE-073, available from FUJIFILM Wako Pure Chemical Corporation), 0.48 g of methacrylic acid (available from Tokyo Chemical Industry Co., Ltd.) and 0.21 g of polyethylene glycol dimethacrylate (n≒4) (available from Tokyo Chemical Industry Co., Ltd.) were added to the above-mentioned ethanol solution while maintaining the temperature of the mixture to 20°C or lower. A mixture containing all of the above, which had been sufficiently stirred and became uniform, was charged in a three-necked flask equipped with a cooling tube, subjected to nitrogen flowing, and the temperature was raised to the reflux temperature while stirring. By heating and stirring the mixture in the state maintaining the above-mentioned environment for 4 hours, a polymer was obtained as a reaction product. The reaction product was reprecipitated with hexane, which is a poor solvent, and the precipitate was collected by filtration and dried under reduced pressure. The obtained powder was dissolved in pure water and the solution was transferred to a dialysis tube. Dialysis was carried out for 72 hours to purify the reaction product.

The solution containing the reaction product was filtered through a 1.0 µm filter (manufactured by AS ONE Corporation, model number: SYGF0605MNXX104) made of a glass fiber, and the obtained filtrate was freeze-dried to obtain a polymer. The weight average molecular weight of this polymer according to GFC was 770,000, and the polydispersity was 4.1 (hereinafter, it is referred to as "Synthetic Example polymer 2").

### <Synthetic Example 3> Production (3) of polymer used for coating film-forming agent of cell culture by thermal polymerization

To 10.00 g of 2-(dimethylamino)ethyl methacrylate (available from Tokyo Chemical Industry Co., Ltd.) was added 31.46 g of ethanol, and the mixture was sufficiently dissolved. Then, 0.01 g of dimethyl 1,1'-azobis(1-cyclohexanecarboxylate) (VE-073, available from FUJIFILM Wako Pure Chemical Corporation), 0.48 g of methacrylic acid (available from Tokyo Chemical Industry Co., Ltd.), 0.26 g of acrylamide (available from Tokyo Chemical Industry Co., Ltd.) and 0.21 g of polyethylene glycol dimethacrylate (n≒4) (available from Tokyo Chemical Industry Co., Ltd.) were added to the above-mentioned ethanol solution while maintaining the temperature of the mixture to 20°C or lower. A mixture containing all of the above, which had been sufficiently stirred and became uniform, was charged in a three-necked flask equipped with a cooling tube, subjected to nitrogen flowing, and the temperature was raised to the reflux temperature while stirring. By heating and stirring the mixture in the state maintaining the above-mentioned environment for 4 hours, a polymer was obtained as a reaction product. The reaction product was reprecipitated with hexane, which is a poor solvent, and the precipitate was collected by filtration and dried under reduced pressure. 0.1 g of the obtained powder was dissolved in 9.9 mL of pure water. 0.003 g of 1-(3-dimethylaminopropyl)-3-ethylcarbodiimide hydrochloride (available from Tokyo Chemical Industry Co., Ltd.) was dissolved in 2.4 mL of pure water, added to the solution prepared above, and stirred and mixed. A mixture dissolved by adding 0.005 g of Alexa Fluor (trademark) 488 Carboxylic Acid, tris(triethylammonium) salt (available from Thermo Fisher Scientific Inc.) to 0.6 mL of dimethylsulfoxide (available from Junsei Chemical Co., Ltd.) was added dropwise to the solution prepared as mentioned above. The mixture was stirred at room temperature for 12 hours to promote the reaction. The obtained reaction solution was transferred to a dialysis tube. Dialysis was carried out for 72 hours to purify the reaction product.

The solution containing the reaction product was filtered through a 1.0 µm filter (manufactured by AS ONE Corporation, model number: SYGF0605MNXX104) made of a glass fiber, and the obtained filtrate was freeze-dried to obtain a polymer. The weight average molecular weight of this polymer according to GFC was 900,000, and the polydispersity was 5.5 (hereinafter, it is referred to as "Synthetic Example polymer 3").

### <Test Example 1> (Composition analysis by ¹H-NMR measurement of polymer)

The nuclear magnetic resonance spectra (NMR) of Synthetic Example polymers 1 to 3 were measured using a nuclear magnetic resonance apparatus (manufactured by BRUKER Corporation, model number: ASCEnd500), and heavy water (D₂O) as a standard substance. In the following, representative peaks common to Synthetic Example polymers 1 to 2 are shown.

¹H-NMR (in D₂O) δ 0.8-1.2 (br, -CH2-C(CH3)-), 1.6-2.0 (br, -CH2-C(CH3)-), 2.2-2.4 (br, -N(CH3)2), 2.5-2.7 (br, -CH2-N(CH3)2), 4.0-4.2 (br, -O-CH2-).

Here, from the number of protons (in the case of the homopolymer of DMAEMA, 3 per one molecule of the monomer) A of the methyl group -CH2-C(CH3)-(δ 0.8-1.2) in the main chain, and the number of methyl protons (in the case of the homopolymer of DMAEMA, 2 per one molecule of the monomer) B of the -O-CH2-group (δ 4.0-4.2) in the side chain, the ratio of the number of the functional groups of the amino groups possessed by the side chain and the number of the functional groups of the carboxyl groups of the side chain was calculated.

As a result, it became 89/11 in the case of Synthetic Example 1, 90/10 in the case of Synthetic Example 2, and 84/16 in the case of Synthetic Example 3. Detailed results are shown in Table 1.
With regard to the polymers synthesized in Synthetic Examples 1 to 3, the weight average molecular weight Mw and molecular weight distribution (PDI: Mw/Mn) measured by GFC are shown in Table 1.

**[Table 1]**

| | Charged ratio | Compositional ratio (**NMR in D2O**) | **Mw (× 10^4)** | **Mw/Mn** |
|---|---|---|---|---|
| | **DM/MA/PEGDMA** | **DM/MA** | | |
| Synthetic Example 1 | **92/8/2** | **89/11** | **66** | **3.8** |
| Synthetic Example 2 | **92/8/2** | **90/10** | **77** | **4.1** |
| Synthetic Example 3 | **92/8/2** | **84/16** | **90** | **5.5** |

### <Example 1: Formation of coating film by inkjet and control of diameter>

### (1) Preparation of cell low-adhesion petri dish

According to the producing method described in Example 30 of WO2014/196650, a coating solution was prepared from a copolymer-containing varnish. The prepared coating solution was added to ϕ 40 mm Asnol petri dish (manufactured by AS ONE Corporation, #1-8549-01) in an amount of 500 µL each, and after allowing to stand at room temperature for one hour, the excess coating solution was removed. Thereafter, 2 mL of sterilized water was added per one well, and then, removed to carry out washing thereof. Similarly, washing was further carried out twice, and it was dried at room temperature for 30 minutes to obtain a cell low-adhesion petri dish.

### (2) Formation of coating film by inkjet and control of diameter

Synthetic Example polymer 3 was so dissolved with sterilized water that the concentration became 100 ng/µL to produce a coating film-forming agent 3 for cell culture. Using an inkjet device (manufactured by MICROJET Co., Ltd., model number: LaboJet-600) and an inkjet head (model number: 500-S-C), the coating film-forming agent 3 was coated onto the cell low-adhesion petri dish prepared above in a spot shape while controlling an amount of droplets (number of ejected droplets) with 55 nL, 165 nL, 330 nL, 660 nL and 1320 nL. Five or more points were coated at each coating amount. They were dried at room temperature for 30 minutes to form a film. 2 mL of pure water was added, and then, removed to carry out washing thereof. Similarly, washing was further carried out twice, and it was dried at room temperature for 30 minutes to obtain a petri dish for forming cell aggregates. The obtained petri dish was observed with fluorescence with a fluorescence microscope (manufactured by Olympus Corporation: model number IX-83) to confirm whether a film was formed or not. The results are shown in Fig. 1(a).

As shown in Fig. 1(a), the portion coated with the coating film-forming agent shines in a circle, and it was shown that a coating film (spot) can be formed by inkjet. Also, as shown in Fig. 1(b) and Table 2, as the amount of droplets increased, the diameter of the circle of the coating film increased. It was shown that the size of the coating film in a spot shape can be controlled by applying the cell culture underground material using the inkjet device.

**[Table 2]**

| Coated amount (nL) | Diameter (mm) | Area (mm²) |
|---|---|---|
| 55 | 0. 84 | 0. 55 |
| 165 | 1. 03 | 0. 83 |
| 330 | 1. 35 | 1. 43 |
| 660 | 1. 77 | 2.46 |
| 1320 | 2. 22 | 3. 87 |

### <Example 2: Formation of high-density coating film by inkjet and determination of spot density>

Synthetic Example polymer 3 was so dissolved with sterilized water that the concentration became 100 ng/µL to produce a coating film-forming agent 3 for cell culture. Using an inkjet device (manufactured by MICROJET Co., Ltd., model number: LaboJet-600) and an inkjet head (model number: IJHB-1000), the coating film-forming agent 3 was coated in a spot shape to the cell low-adhesion petri dish prepared in Example 1(1) with each 950 pL with a distance of 300 µm. It was dried at room temperature for 30 minutes to form a film. 2 mL of pure water was added, and then, removed to carry out washing thereof. Similarly, washing was further carried out twice, and it was dried at room temperature for 30 minutes to obtain a petri dish for forming cell aggregates coated at high-density. The obtained petri dish was observed with fluorescence with a fluorescence microscope (manufactured by Olympus Corporation: model number IX-83).

As shown in Fig. 2(a), the portion coated with the coating film-forming agent shines in a circle, and it was shown that a coating film (spot) can be formed by inkjet with high-density. A ratio of the total area of the spots to the bottom surface area of the petri dish was 44%.

### <Comparative Example 1: Formation of low-density coating film by inkjet and determination of spot density >

Synthetic Example polymer 3 was so dissolved with sterilized water that the concentration became 100 ng/µL to produce a coating film-forming agent 3 for cell culture. Using an inkjet device (manufactured by MICROJET Co., Ltd., model number: LaboJet-600) and an inkjet head (model number: 500-S-C), the coating film-forming agent 3 was coated in a spot shape to the cell low-adhesion petri dish prepared in Example 1(1) with each 55 nL with a distance of 2 mm. It was dried at room temperature for 30 minutes to form a film. 2 mL of pure water was added, and then, removed to carry out washing thereof. Similarly, washing was further carried out twice, and it was dried at room temperature for 30 minutes to obtain a petri dish for forming cell aggregates. The obtained petri dish was observed with fluorescence with a fluorescence microscope (manufactured by Olympus Corporation: model number IX-83).

As shown in Fig. 2(b), the portion coated with the coating film-forming agent 3 shines in a circle, and it was shown that a coating film (spot) can be formed by inkjet. A ratio of the total area of the spots to the bottom area of the petri dish was 15%.

### <Example 3: Production test of size-controlled cell aggregates>

### (1) Preparation of petri dish for forming cell aggregates

Synthetic Example polymer 2 was so dissolved with sterilized water that the concentration became 100 ng/µL to produce a coating film-forming agent 2 for cell culture. Using an inkjet device (manufactured by MICROJET Co., Ltd., model number: LaboJet-600) and an inkjet head (model number: 500-S-C), the coating film-forming agent 2 was coated onto the cell low-adhesion petri dish prepared in Example 1(1) in a spot shape while controlling an amount of droplets (number of ejected droplets) with 55 nL, 165 nL, 330 nL, 660 nL and 1320 nL. Five or more points were coated at each coating amount. It was dried at room temperature for 30 minutes to form a film. 2 mL of pure water was added, and then, removed to carry out washing thereof. Similarly, washing was further carried out twice, and it was dried at room temperature for 30 minutes to obtain a petri dish for forming cell aggregates.

### (2) Preparation of cells

Mouse fetal fibroblasts (C3H10T1/T2 cells: available from DS Pharma Biomedical Co., Ltd.) were used as cells. For culturing the cells, a medium in which, to a BME medium (available from Gibco) which became a basal medium, were added so as to be 10% of FBS (available from Sigma-Aldrich) and 1% of Glutamine/Penicillin/Streptomycin (available from Gibco) was used. The cells were statically cultured for 2 days or more in a petri dish (medium 10 mL) having a diameter of 10 cm while maintaining a 5% carbon dioxide concentration in a 37°C/CO₂ incubator. Subsequently, the present cells were washed with 3 mL of a PBS solution (available from FUJIFILM Wako Pure Chemical Corporation), then, 3 mL of a trypsin-EDTA solution (available from PromoCell GmbH) was added thereto, and the cells were allowed to stand at room temperature for 3 minutes to exfoliate the cells. The cells were recovered by adding 7 mL of the above-mentioned medium. This suspension was centrifuged (manufactured by TOMY SEIKO Co., Ltd., model number LC-230, 200 × g/3 min, room temperature), and then, the supernatant was removed, and the above-mentioned medium was added to prepare a cell suspension.

### (3) Cell adhesion experiment

To the petri dish prepared in the above-mentioned (1) was added 500 µL of each cell suspension so that it became 3.0×10⁵ cells/cm². Thereafter, it was allowed to stand in a 37°C/CO₂ incubator for 4 hours while maintaining a 5% carbon dioxide concentration. After allowing to stand, non-adherent cells and the medium were removed, and it was washed with PBS to leave adherent cells alone on the wells. After washing, 500 µL/well of a new medium was added thereto, and the state of adherent cells was observed and photographed using an inverted research microscope IX83 (manufactured by Olympus Corporation). As a result, as shown in Fig. 3(a), adhesion of cells to the portion (spot) on which the coating film-forming agent 2 for cell culture had been coated was confirmed. Further, it could be found that when an amount of droplets increased, the adhesion area of the cells also increased.

### (4) Observation of cell aggregates

The petri dish tested above was allowed to stand in a 37°C/CO₂ incubator for further 44 hours. After allowing to stand, the state of the cells was observed using an inverted research microscope IX83 (manufactured by Olympus Corporation). As a result, as shown in Fig. 3(a), it was confirmed that the cells adhered to the coating film-forming agent 2 for cell culture were exfoliated from the petri dish and aggregated to form a cell aggregate (spheroid).

In addition, the relationship between the coated amount, the coated area and the diameter of the spheroid is shown in Fig. 3(b) and Table 3. It was shown that when the coated amount became larger, the coated area was larger, and the size of the obtained spheroid became large. In addition, when it was evaluated with n=5, the size error became 3.5 to 8.4%. From this result, it was shown that the coating film containing the polymer of the present invention is useful as a coating film for forming spheroids of any optional size with a small size error by controlling the coated amount in combination with an inkjet device.

**[Table 3]**

| Coated amount (nL) | Coated area (mm²) | Error (mm²) | Average value (µm) of spheroid diameter Error | Error (*µ*m) | Error (%) |
|---|---|---|---|---|---|
| 55 | 0.5 | 0. 09 | 181 | 11 | 6. 0 |
| 165 | 1. 07 | 0. 03 | 250 | 10 | 3.8 |
| 330 | 1. 91 | 0. 19 | 327 | 27 | 8. 4 |
| 660 | 3.41 | 0.21 | 368 | 13 | 3. 5 |
| 1320 | 5.6 | 0.47 | 449 | 27. 4 | 6. 1 |

### <Example 4: Cell adhesion confirmation test by high-density coating>

### (1) Preparation of petri dish for forming cell aggregates

Synthetic Example polymer 2 was so dissolved with sterilized water that the concentration became 100 ng/µL to produce a coating film-forming agent 2 for cell culture. Using an inkjet device (manufactured by MICROJET Co., Ltd., model number: LaboJet-600) and an inkjet head (model number: IJHB-1000), the coating film-forming agent 2 was coated in a spot shape to the cell low-adhesion petri dish prepared in Example 1(1) with each 900 to 950 pL with a distance of 300 µm. It was dried at room temperature for 30 minutes to form a film. 2 mL of pure water was added, and then, removed to carry out washing thereof. Similarly, washing was further carried out twice, and it was dried at room temperature for 30 minutes to obtain a petri dish for forming cell aggregates coated at high-density. A ratio of the total area of the spots to the bottom surface area of the petri dish was 52%.

### (2) Cell adhesion experiment

To the petri dish prepared in the above-mentioned (1) was added 500 µL of the cell suspension prepared in Example 3(1) so that it became 3.0×10⁵ cells/cm². Thereafter, it was allowed to stand in a 37°C/CO₂ incubator for 4 hours while maintaining a 5% carbon dioxide concentration. Without subjecting to washing step, the state of adherent cells was observed and photographed using an inverted research microscope IX83 (manufactured by Olympus Corporation). As a result, as shown in Fig. 4(a), adhesion of cells to the portion (spot) on which the coating film-forming agent 2 for cell culture had been coated was confirmed. In addition, there were no cells floating without adhesion

### <Comparative Example 2: Cell adhesion confirmation test by low-density coating film>

### (2-1. Preparation of petri dish for forming cell aggregates)

Synthetic Example polymer 2 was so dissolved with sterilized water that the concentration became 100 ng/µL to produce a coating film-forming agent 2 for cell culture. Using an inkjet device (manufactured by MICROJET Co., Ltd., model number: LaboJet-600) and an inkjet head (model number: 500-S-C), the coating film-forming agent 2 was coated in a spot shape to the cell low-adhesion petri dish prepared in Example 1(1) with each 55 nL with a distance of 2 mm. It was dried at room temperature for 30 minutes to form a film. 2 mL of pure water was added, and then, removed to carry out washing thereof. Similarly, washing was further carried out twice, and it was dried at room temperature for 30 minutes to obtain a petri dish for forming cell aggregates. A ratio of the total area of the spots to the bottom surface area of the petri dish was 15%.

### (2) Cell adhesion experiment

To the petri dish prepared in the above-mentioned (1) was added 500 µL of the cell suspension prepared in Example 3(1) so that it became 3.0×10⁵ cells/cm². Thereafter, it was allowed to stand in a 37°C/CO₂ incubator for 2 hours while maintaining a 5% carbon dioxide concentration. Without subjecting to washing step, the state of adherent cells was observed and photographed using an inverted research microscope IX83 (manufactured by Olympus Corporation). As a result, as shown in Fig. 4(b), adhesion of cells to the portion (spot) on which the coating film-forming agent 2 for cell culture had been coated, and many other floating cells were confirmed.

### <Example 5: Cell aggregates production test by high-density coating>

### (1) Preparation of petri dish for forming cell aggregates

Synthetic Example polymer 1 was so dissolved with sterilized water that the concentration became 100 ng/µL to produce a coating film-forming agent 1 for cell culture. Using an inkjet device (manufactured by MICROJET Co., Ltd., model number: LaboJet-600) and an inkjet head (model number: IJHB-1000), the coating film-forming agent 1 was coated in a spot shape to the cell low-adhesion petri dish prepared in Example 1(1) with each 950 pL with a distance of 300 µm. It was dried at room temperature for 30 minutes to form a film. 2 mL of pure water was added, and then, removed to carry out washing thereof. Similarly, washing was further carried out twice, and it was dried at room temperature for 30 minutes to obtain a petri dish for forming cell aggregates coated at high-density.

### (2) Cell adhesion experiment

To the petri dish prepared in the above-mentioned (1) was added 500 µL of the cell suspension prepared in Example 3(1) so that it became 3.0×10⁵ cells/cm². Thereafter, while maintaining a 5% carbon dioxide concentration, the states of adherent cells and aggregation were observed while culturing with a time lapse using an inverted research microscope IX83 (manufactured by Olympus Corporation). As a result, as shown in Fig. 5, it was confirmed that the cells dispersed over the entire surface immediately after seeding adhered to the portion (spot) on which the coating film-forming agent 1 for cell culture had been coated with a lapse of time. At the time after 2 hours, there were no cells present floating without adhesion. Further, at the time after 8 hours, it was confirmed that the adhered cells were exfoliated from the petri dish and aggregated to form cell aggregates (spheroids).

### UTILIZABILITY IN INDUSTRY

From the above, it could be shown that all the seeded cells could be adhered to the spot and aggregated by applying the coating film containing the polymer according to the present invention in a high-density and spot shape to the substrate (for example, the cell low-adhesion petri dish) having an ability to inhibit cell adhesion, in combination with an inkjet device. That is, without using a multi-well plate for forming spheroids, it could be shown that cells are merely seeded on the plane surface of the substrate having a coating film (spots), not only spheroids with a uniform size can be formed, but also utilization efficiency of the cells seeded at the time of producing the spheroids is improved by using the substrate for producing cell aggregate of the present invention, which is provided with a plurality of spots comprising the polymer according to the present invention with high-density on a substrate having an ability to inhibit cell adhesion such as a bottom surface of a petri dish or a dish or a plate surface.

## Claims

1. A substrate for producing cell aggregates provided with a plurality of spots which comprises a polymer containing a recurring unit derived from a monomer represented by the following formula (I): 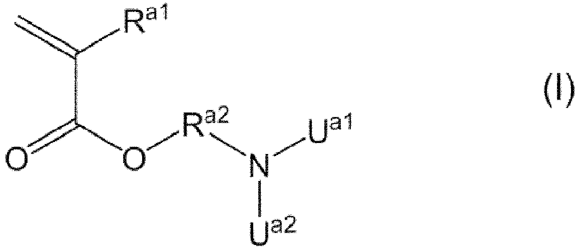 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
on a substrate having an ability to inhibit cell adhesion, wherein a ratio of a total area of the spots to a surface area of the substrate is 30% or more, a diameter of each spot is 50 to 5,000 µm, and a distance between the spots is 30 to 1,000 µm.

2. The substrate for producing cell aggregates according to claim 1, wherein cell utilization efficiency is 90% or more.

3. The substrate for producing cell aggregates according to claim 1 or 2, wherein the cell is a stem cell.

4. The substrate for producing cell aggregates according to any one of claims 1 to 3, wherein the polymer further contains a recurring unit derived from a monomer represented by the formula (II): 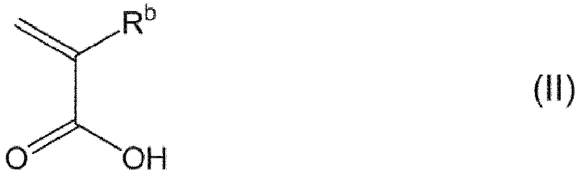 wherein
R^{b} represents a hydrogen atom or a linear or branched alkyl group having 1 to 5 carbon atoms.

5. The substrate for producing cell aggregates according to any one of claims 1 to 4, wherein the polymer further contains a structural unit derived from a monomer represented by the following formula (III): 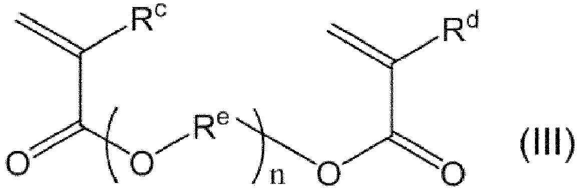 wherein
R^{c} and R^{d} each independently represent a hydrogen atom or a linear or branched alkyl group having 1 to 5 carbon atoms, R^{e} represents a linear or branched alkylene group having 1 to 5 carbon atoms, and n represents a number of 1 to 50.

6. The substrate for producing cell aggregates according to any one of claims 1 to 5, wherein the substrate having an ability to inhibit cell adhesion comprises a coating film which comprises a copolymer (P) having a recurring unit containing a group represented by the following formula (a) and a recurring unit containing a group represented by the following formula (b): 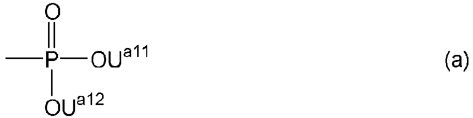 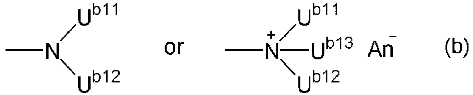 wherein
U^{a11}, U^{a12}, U^{b11}, U^{b12} and U^{b13} each independently represent a hydrogen atom or a linear or branched alkyl group having 1 to 5 carbon atoms, An⁻ represents an anion selected from the group consisting of a halide ion, an inorganic acid ion, a hydroxide ion and an isothiocyanate ion,
at least a part of a surface of the substrate.

7. A method for producing a substrate for producing cell aggregates which comprises a step of applying a polymer containing a recurring unit derived from a monomer represented by the following formula (I): 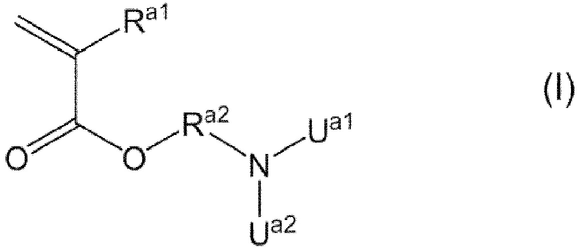 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
to a substrate having an ability to inhibit cell adhesion in a state of a plurality of spots, and then, drying,
wherein a total area of the spots based on a surface area of the substrate is 30% or more, a diameter of each spot is 50 to 5,000 µm, and a distance between the spots is 30 to 1,000 µm.

8. The method for producing a substrate for producing cell aggregates according to claim 7, wherein cell utilization efficiency is 90% or more.

9. The method for producing a substrate for producing cell aggregates according to claim 7 or 8, wherein the applying step is carried out by an inkjet system.

10. A method for producing cell aggregates which comprises a step of applying a polymer containing a recurring unit derived from a monomer represented by the following formula (I): 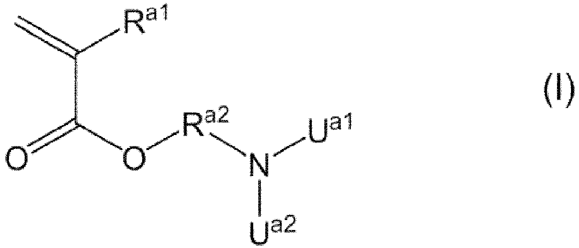 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
to a substrate having an ability to inhibit cell adhesion in a state of a plurality of spots, and then, a step of seeding cells,
wherein a total area of the spots based on a surface area of the substrate is 30% or more, a diameter of each spot is 50 to 5,000 µm, and a distance between the spots is 30 to 1,000 µm.

11. The method for producing cell aggregates according to claim 10, wherein cell utilization efficiency is 90% or more.

12. A method for improving cell utilization efficiency at a time of producing cell aggregates by applying a polymer containing a recurring unit derived from a monomer represented by the following formula (I): 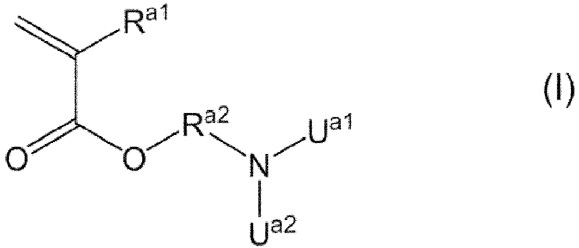 wherein
U^{a1} and U^{a2} each independently represent a hydrogen atom or a linear or branched alkyl group having 1 to 5 carbon atoms, R^{a1} represents a hydrogen atom or a linear or branched alkyl group having 1 to 5 carbon atoms, and R^{a2} represents a linear or branched alkylene group having 1 to 5 carbon atoms,
to a substrate having an ability to inhibit cell adhesion in a state of a plurality of spots, wherein a total area of the spots based on a surface area of the substrate is 30% or more, a diameter of each spot is 50 to 5,000 µm, and a distance between the spots is 30 to 1,000 µm.
